# EUROPEAN PATENT SPECIFICATION

(11) **EP 0 686 382 B1**
(45) Date of publication and mention of the grant of the patent: **19.12.2001**
(21) Application number: 95500080.7
(22) Date of filing: 06.06.1995
(51) Int. Cl.: A61F 6/14

(54) **Intrauterine device**
Intrauterinvorrichtung
Dispositif intra-utérine

(30) Priority: 07.06.1994 ES 9401573 U
(43) Date of publication of application: 13.12.1995
(73) Proprietor: Compania de Implantes Clinicos, S.A., 28046 Madrid (ES)
(72) Inventor: Foppiano, Marco C., E-28003 Madrid (ES)
(74) Representative: Lehmann Novo, Maria Isabel

(56) References cited:
- EP-A- 0 307 498
- WO-A-91/07934
- DE-A- 3 209 290
- FR-A- 2 529 079

## Description

### SUBJECT OF THE INVENTION

This invention refers to an intrauterine device incorporating a pull out system as per the preamble of claim 1. It is suitable for placing in an insertion tube or pipe, provided with certain characteristics giving notable advantages in comparison with intrauterine devices known up to now.

This invention has been conceived and structured with the purpose of achieving an improvement in the manoeuvring prior to inserting the intrauterine device, enabling its volume to be reduced and, consequently, to ease its passage or penetration through the uterine cervical canal.

### BACKGROUND TO THE INVENTION

Pessaries or intrauterine devices, also known as IUDs, are generally structured on the basis of an inert, usually T, V or Y shaped plastic support on whose vertical centre branch or shaft is wound a copper coil so that the overall device suitably fitted into the uterus body defines a strategic siting of the copper mass or masses constituting a spermicide barrier. These devices must be periodically replaced and, in any event, withdrawn from the uterine cavity. To this effect, they have an orifice in the bottom end of the vertical shaft through which a cord passes, commonly known as the pull cord. With the right length, this cord is the pull or drag element during the manoeuvre loading the intrauterine device into the insertion tube or pipe and subsequently during withdrawal of the device from the uterine cavity.

The horizontal arms or branches of these types of intrauterine devices are elastically deformable so that during the positioning manoeuvre, both arms join to each other within an insertion tube or pipe provided with an axial pusher made up of a plunge applicator whilst, when being withdrawn, these arms close on being dragged out by the thread.

From WO-A-91/07934 an intrauterine device is known that comprises a vertical middle leg or shaft and two cross legs or arms starting from the top end of the shaft, such arms being able to fold down to become an extension of the shaft when the device is placed in an insertion tube or pipe. The shaft has an orifice at its top end, near the point where the arms start, through which fits a pulling thread, and a narrowing near its bottom end, followed by a final part. The structure of the device is completed by a coil or spring fitted over the shaft having spermicide properties.

This structuring gives rise to the following problem in practice: pulling for locating the intrauterine device in the insertion tube or pipe requires a certain amount of force which, depending on the hardness of the plastic material of which the device body is made, may reach 10 Nw. This is why these types of intrauterine devices thicken at their bottom end more than the diameter of their vertical shaft so as to prevent the latter breaking when pulling the cord. This has the disadvantage of the insertion tube or pipe having to have a diameter more than necessary which causes greater discomfort to the patient or user when the tube or pipe penetrates through the uterine cervical canal.

### SUMMARY OF THE INVENTION

There is therefore a need for an intrauterine device fitted with a cord pull system conceived and structured so that a completely satisfactory solution to the aforementioned problems is provided. This is where this invention has a place. Starting from the basic structuring of a conventional intrauterine pessary with a thickening of the vertical shaft's bottom end where the thread is located, this invention offers the characteristic consisting of the thread pass-through orifice being located in the top end or close to the point where its elastically deformable horizontal arms start. In addition, this vertical shaft embodies two diametrically opposite longitudinal grooves for accommodating the two lengths of cord. The latter are located between the vertical shaft and the copper coil. Thus the full distancing of this orifice from the free end of the vertical shaft makes it impossible for the latter to break during the pull, without the need to increase the diameter of this intrauterine device's vertical shaft at any point and thus allowing a lesser diameter insertion pipe to be used.

The essential features of the intrauterine device of the invention are fully described in the characterizing portion of claim 1 while further supplementary features are shown in dependent claims 2 and 3.

### BRIEF DESCRIPTION OF THE DRAWINGS

In order to complete the description of the invention's intrauterine device, this descriptive section is accompanied by a set of drawings forming an integral part thereof. Such drawings show the following which, whilst illustrative in nature, is not limiting:
- In figure 1,: one of the several T-shaped models of intrauterine devices already existing in which the bottom end of the vertical shaft displays a sphere shaped thickening larger in diameter than the said shaft, where the thread is located.
- In figure 2,: an intrauterine device incorporating the improved characteristics of this invention.
- In figure 3,: a cross section through A-A' of figure 2, and
- In figure 4,: a detail of the copper cap finishing off the bottom end of the vertical shaft of the device shown in figure 2.

### DETAILED DESCRIPTION OF THE INVENTION

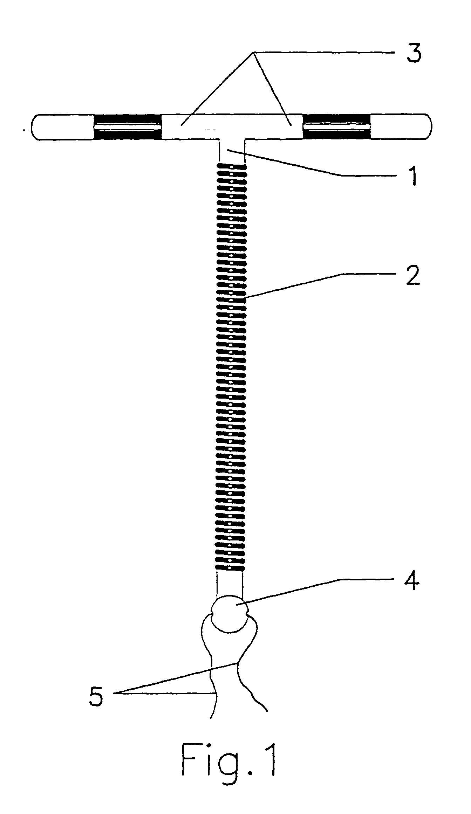

As can be seen in figure 1, a conventional intrauterine device comprises a shaft 1 on which a wire spermicide coil or spring of copper or metal alloy 2 is wound. The shaft 1 ends at the top in two arms 3 and at the bottom in a thickening 4 which is greater in diameter than the shaft 1 and acts as an anchoring for the thread 5. The diameter of the intrauterine device's insertion tube or pipe will therefore have to be greater than the diameter of the shaft 1 and at least equal to the diameter of the thickening 4.

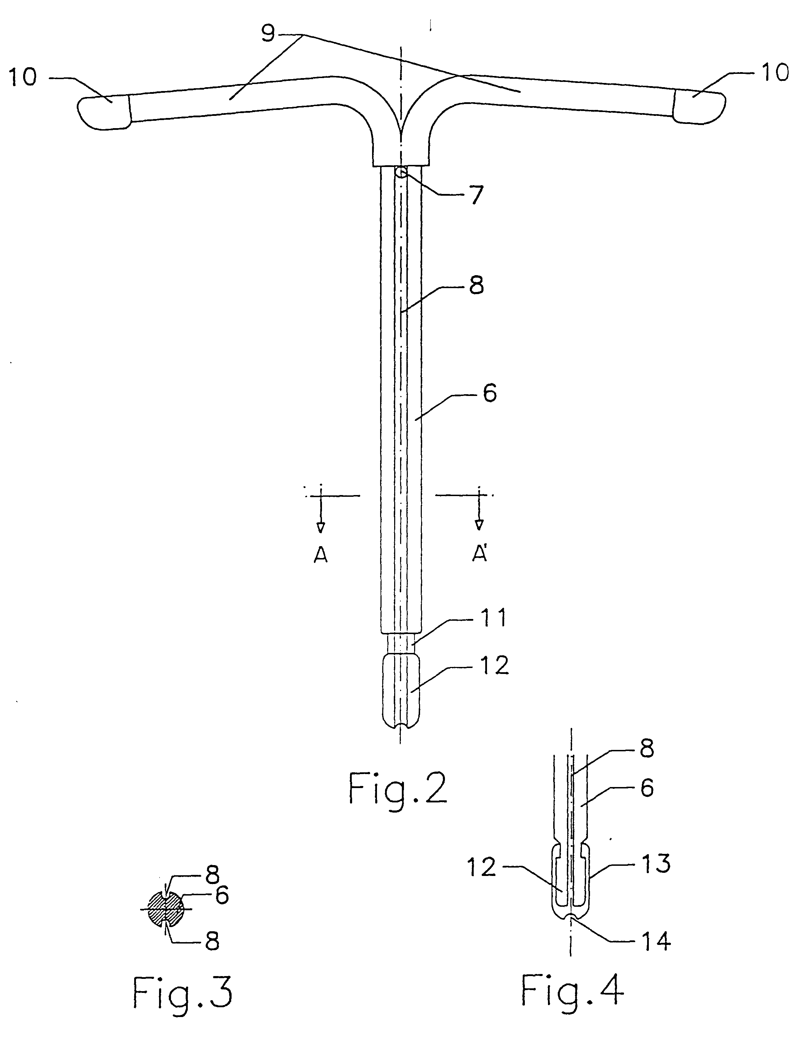

On the other hand, no thickening of the bottom end of the shaft is required in the invention's intrauterine device illustrated in figures 2 to 4, since the force produced by the thread is not exerted on the said bottom end but is distributed along the shaft. To this end, the invention's intrauterine device displays an orifice 7 in its central vertical shaft for the thread (not shown) to pass through, as well as two diametrically opposite, longitudinal grooves 8 to which the two lengths of thread passing through the orifice 7 adapt so that the said lengths of thread are within the vertical shaft 6 and underneath the copper coil (not shown). These longitudinal grooves 8 can be clearly seen in the cross section view of figure 3.

As a result of the way in which the pull cord is arranged in the invention's intrauterine device, it is practically impossible for the vertical shaft 6 to break whilst the pull cord is being pulled and there is no need to increase its diameter anywhere. This enables an insertion pipe with a smaller diameter than in conventional intrauterine devices to be used and discomfort caused to the patient or user of the device when placing into the cavity of the uterus to be reduced.

The intrauterine device shown in figure 2 has two arms 9 with a channel shaped cross section having thickenenings 10 at their ends which will be pushed against the uterus wall after the intrauterine device is placed in position. Nevertheless, it is understood that the arms 9 do not necessarily have to be as shown in figure 2, since the invention's intrauterine device could, for example, have circular cross section arms with no end thickening. Therefore, the important thing in the invention's intrauterine device is not the configuration of its arms but the presence of orifice 7 near the point where such arms start and the existence of two diametrically opposite longitudinal grooves 8, each of which communicates with one of the mouths of the said orifice 7.

The vertical shaft 6 of this invention's intrauterine device shows a narrowing 11 near its bottom end. The shaft is continued along an end part 12 with a diameter somewhat larger than the said narrowing's 11, but small than the rest of the shaft 6. A copper cap 13 is fitted onto this end part 12 (see figure 4). The cap has an orifice 14 in the centre of its bottom end through which the thread appears. The cap 13 has a diameter the same as the part of the shaft 6 extending above the aforesaid narrowing 11. Therefore, the insertion tube or pipe used for positioning the intrauterine device may have a diameter noticeably the same as the said shaft's 6 which, as has already been said, will reduce discomfort caused to the patient or user of the intrauterine device when the insertion tube or pipe penetrates through the uterine cervical canal.

The foregoing describes the intrauterine device's characteristics deemed essential in accordance with this invention. Nevertheless, it is understood that an expert could make certain detail modifications to this device, like those already indicated in relation to its arms or of some other kind. However, such modifications would be in the scope of this invention as defined in the attached claims.

## Claims

1. Intrauterine device of the type comprising a central vertical shaft (6) and two arms (9) starting from the top end of the shaft (6), whereby such arms (9) may fold down to become an extension of the shaft (6) when the device is placed in an insertion tube or pipe, said central shaft (6) having an orifice (7) at its top end, near the point where the arms (9) start, through which fits a thread (5), and a narrowing (11) near its bottom end, followed by a final part (12), the intrauterine device further comprising a coil or spring (2) fitted over the shaft (6) having spermicide properties, **characterized in that** said central shaft (6) has two diametrically opposite longitudinal grooves (8) which are occupied by two lengths of the thread (5) passing through the orifice (7), these lengths being located underneath said spermicide coil or spring (2) and the final part (12) being covered by a cap (13) provided with a central orifice (14) where said thread (5) appears.

2. Device according to claim 1, **characterized in that** the arms (9) have a channel shaped cross section and end in two bulges (10).

3. Device ccording to either of the preceding claims, **characterized in that** the said coil (2) and the said cap (13) are copper or a metal alloy with spermicide properties.

## Patentansprüche

1. Intrauterinpessar jener Art, die einen mittleren vertikalen Schaft (6) und zwei vom oberen Ende des Schafts (6) ausgehende Arme (9) umfaßt, wodurch solche Arme (9) nach unten faltbar sind und so zu einer Verlängerung des Schafts (6) werden, wenn die Vorrichtung in einer Einführröhre oder einem Einführrohr angeordnet wird, wobei der mittlere Schaft (6) an seinem oberen Ende in der Nähe des Ausgangspunkts der Arme (9) eine Öffnung (7), durch die ein Faden (5) paßt, und in der Nähe seines unteren Endes eine Verengung (11) aufweist, an die sich ein Endteil (12) anschließt, wobei das Intrauterinpessar weiterhin eine Spirale oder eine Feder (2) umfaßt, die über den Schaft (6) angeordnet ist und spermizide Eigenschaften aufweist, **dadurch gekennzeichnet, daß** der mittlere Schaft (6) zwei diametral gegenüberliegende Längsnuten (8) enthält, die von zwei Längen des durch die Öffnung (7) geführten Fadens (5) eingenommen werden, wobei diese Längen unterhalb der spermiziden Spirale oder Feder (2) angeordnet sind und der Endteil (12) durch eine Kappe (13) bedeckt wird, die mit einer mittleren Öffnung (14) versehen ist, aus der der Faden (5) hervortritt.

2. Vorrichtung nach Anspruch 1, **dadurch gekennzeichnet, daß** die Arme (9) einen kanalförmigen Querschnitt aufweisen und in zwei Ausbauchungen (10) abschließen.

3. Vorrichtung nach einem der vorhergehenden Ansprüche, **dadurch gekennzeichnet, daß** die Spirale (2) und die Kappe (13) eine Kupfer- oder Metallegierung mit spermiziden Eigenschaften sind.

## Revendications

1. Dispositif intra-utérin du type comprenant une tige centrale verticale (6) et deux bras (9) partant de l'extrémité supérieure de la tige (6), ces bras (9) pouvant se replier pour devenir une extension de la tige (6) lorsque le dispositif est placé dans un tuyau ou tube d'insertion, ladite tige centrale (6) ayant un orifice (7) au niveau de son extrémité supérieure, près du point d'où partent les bras (9), à travers lequel orifice passe un fil (5), et un rétrécissement (11) près de son extrémité inférieure, suivi d'une partie finale (12), le dispositif intra-utérin comprenant en outre un serpentin ou un ressort (2) ajusté par-dessus la tige (6) ayant des propriétés spermicides, **caractérisé en ce que** ladite tige centrale (6) a deux rainures longitudinales diamétralement opposées (8) qui sont occupées par deux longueurs du fil (5) passant à travers l'orifice (7), ces longueurs étant situées sous ledit serpentin ou ressort spermicide (2) et la partie finale (12) étant recouverte par un capuchon (13) pourvu d'un orifice central (14) où apparaît ledit fil (5).

2. Dispositif selon la revendication 1,
**caractérisé en ce que** les bras (9) ont une section transversale en forme de canal et se terminent par deux protubérances (10).

3. Dispositif selon l'une ou l'autre des revendications précédentes, **caractérisé en ce que** ledit serpentin (2) et ledit capuchon (13) sont en alliage de cuivre ou de métal avec des propriétés spermicides.
